# EUROPÄISCHE PATENTSCHRIFT

(11) **EP 0 381 841 B1**
(45) Veröffentlichungstag und Bekanntmachung des Hinweises auf die Patenterteilung: **26.05.1993**
(21) Anmeldenummer: 89123117.7
(22) Anmeldetag: 14.12.1989
(51) Int. Cl.: B65B 55/10, B08B 9/20

(54) **Verfahren und Vorrichtung zum Reinigen und Sterilisieren von Behältern**
Method and device for cleaning and sterilizing containers
Procédé et dispositif de nettoyage et stérilisation de récipients

(30) Priorität: 27.01.1989 DE 3902432
(43) Veröffentlichungstag der Anmeldung: 16.08.1990
(73) Patentinhaber: ROBERT BOSCH GMBH, 70442 Stuttgart (DE)
(72) Erfinder: Buchner, Norbert, Prof.-Dr., D-7057 Winnenden 6 (DE); Lemke, Kuno, D 7120 Bietigheim-Bissingen (DE); Voegele, Guenther, D-7036 Schönaich (DE); Weber, Helmut, D-7056 Weinstadt-Endersb. (DE)

(56) Entgegenhaltungen:
- EP-A- 0 187 468
- US-A- 1 940 615
- US-A- 4 099 674

## Beschreibung

### Stand der Technik

Die Erfindung geht aus von einem Verfahren und einer Vorrichtung zum Reinigen und Sterilisieren von Verpackungsbehältern, wie Flaschen, Ampullen und dergleichen nach dem Oberbegriff des Anspruchs 1. Aus der DE-B-12 68 996 ist es schon bekannt, Flaschen durch Spülen mit einem Druckgas, beispielsweise Luft zu säubern, wobei Staub- und Fremdteile herausgeblasen werden. Durch exzentrisches Einführen und Umlenken des Luftstromes am Flaschenboden sollen Turbulenzen vermieden und eine stärkere Strömung erzielt werden. Bei Verwendung eines heißen Gases soll außer dem Ausspülen von Verunreinigungen das Flascheninnere auch sterilisiert werden.

Ferner ist aus der GB-A-969 084 ein Verfahren zum Reinigen und Sterilisieren von Flaschen vor dem Befüllen bekannt, bei dem man die Flaschen zunächst mit Heißwasser spült, darauf mehrere Male mit Wasserdampf beaufschlagt und schließlich das Kondensat abfließen läßt.

### Vorteile der Erfindung

Das erfindungsgemäße Verfahren mit den kennzeichnenden Merkmalen des Anspruchs 1 hat demgegenüber den Vorteil, daß Verunreinigungen vollständig gelöst sowie zusammen mit dem Kondensat entfernt werden, und daß Mikroorganismen, die im ph-Bereich kleiner 4,5 relevant sind, vollständig abgetötet werden. Ferner erfordert das erfindungsgemäße Verfahren einen geringen Aufwand an Dampf- und Druckluftmengen und ist in sehr kurzer Zeitdauer durchführbar. Als sehr wirkungsvoll hat sich die Verwendung von strömendem Wasserdampf bei atmosphärischem Druck während einer Zeitdauer von 2 bis 5 Sekunden und das Abziehen des Kondensats mit Sterilluft während einer Zeitdauer von 1 bis 3 Sekunden erwiesen. Das Verfahren liefert intensiv gereinigte, angewärmte Flaschen, die für die Erfordernisse eines nachgeschalteten weiteren Sterilisierverfahrens, z.B. mit H₂O₂-Dämpfen, vollständig trocken sind.

Für den ph-Bereich größer 4,5 läßt sich das Verfahren ausgestalten durch eine Zusatzbehandlung, wie eine Wiederholung des Bedampfungs-Ausblasvorganges und/oder einen thermischen, physikalischen oder chemischen Sterilisiervorgang.

Die in Anspruch 9 angegebene, in Bezug auf GB-A-969 085 abgegrenzte Vorrichtung zum Durchführen des Verfahrens nach Anspruch 1 ist in ihrem Aufbau einfach und arbeitet sehr verläßlich. Die in den übrigen Ansprüchen angegebenen Maßnahmen geben vorteilhafte Ausgestaltungen und Verbesserungen des angegebenen Verfahrens und der Vorrichtung an.

### Zeichnung

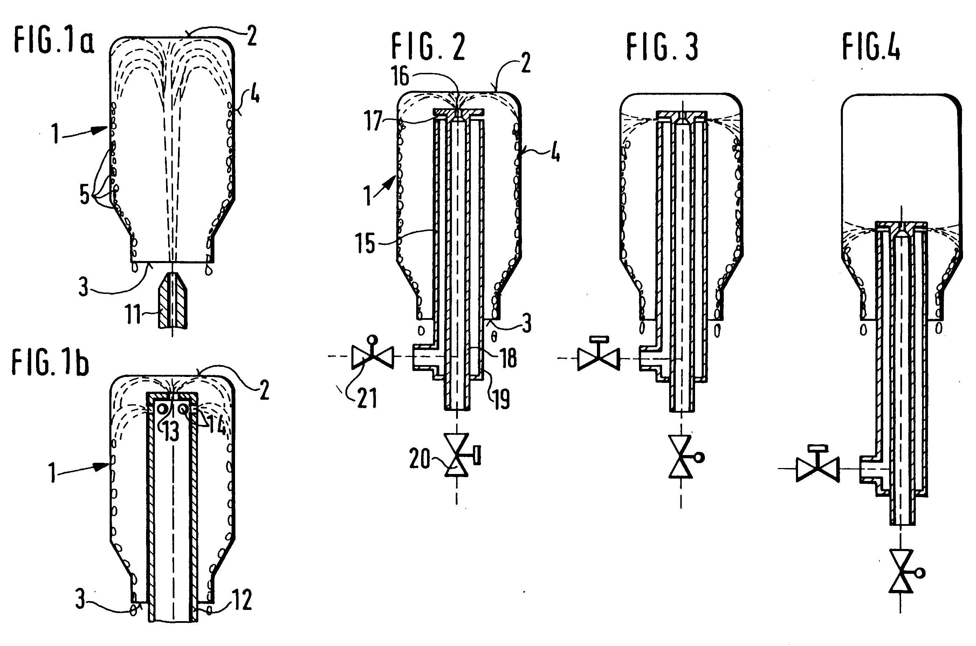

Ein Ausführungsbeispiel der Erfindung ist in der Zeichnung dargestellt und wird im folgenden näher erläutert. Es zeigen Figur 1a und 1b das Bedampfen einer Flasche und Figur 2 bis 4 das Entfernen von Kondensat aus einer Flasche mit einer Düse in mehreren Arbeitsstellungen.

### Beschreibung des Ausführungsbeispiels

Die zu reinigenden Behälter, beispielsweise Flaschen 1, werden zum Behandeln zunächst auf den Kopf gestellt, so daß ihr Boden 2 eine Decke bildet und ihre Einlaßöffnung 3 nach unten weist. Darauf wird durch eine Düse 11 durch die Öffnung 3 Wasserdampf in die Flasche 1 eingeleitet. Der Wasserdampf hat vorzugsweise atmosphärischen Druck (100° C), er kann aber auch überhitzt sein. An der kalten Wandung der Flasche 1 kühlt ein Teil des Wasserdampfes ab und kondensiert zu einem Wasserfilm bestehend aus kleinen Wassertröpfchen 5 (in der Zeichnung sind die Wassertröpfchen 5 der Deutlichkeit wegen übertrieben groß dargestellt). Die Dauer der Einblasung von Wasserdampf beträgt 2 bis 5 Sekunden. Vorzugsweise wird der Wasserdampf mit einer stationären Düse 11 in die Flasche 1 eingeleitet (Figur 1a), über der die zu reinigenden Flaschen 1 der Reihe nach positioniert werden. Alternativ dazu kann auch eine Düse 12 verwendet werden (Figur 1b), die von unten in das Innere der Flasche 1 durch deren Öffnung 3 eingeführt wird, und die eine axiale Öffnung 13 sowie radiale Öffnungen 14 für den Dampfaustritt hat.

Nach dem Bedampfen wird die Flasche 1 auf einer nachfolgenden Station in axiale Ausrichtung mit einer Blasdüse 15 gebracht, die vertikal heb- und senkbar angeordnet ist. Diese als Doppeldüse ausgebildete Blasdüse 15 hat an ihrem Kopf eine axiale Öffnung 16 und darunter einen Ringspalt 17. Der Ringspalt 17 ist, wie in der Zeichnung dargestellt, horizontal ausgerichtet, er kann aber auch etwas geneigt in Form eines Kegelmantels angeordnet sein. Die axiale Öffnung 16 ist durch ein Innenrohr 18 und ein Ventil 20 und der Ringspalt 17 über ein konzentrisches Außenrohr 20 und ein Ventil 21 mit einer nicht dargestellten Druckgas- oder Druckluftquelle verbunden. Vorzugsweise liefert diese Quelle steriles Druckgas bzw. sterile Druckluft.

Nach dem Einführen der Blasdüse 15 in die Flasche 1 in eine obere Stellung, in der sie einen Abstand von etwa 4 bis 10 mm vom Boden 2 hat, wird das Ventil 20 geöffnet, so daß mit etwa 5 bar vorgespannte, sterile Druckluft im Innenraum der Flasche 1 mit hoher Aufprallenergie gegen die Mitte des Bodens 2 bläst (Figur 2). Dabei treibt die am Boden 2 umgelenkte Luftströmung das am Boden 2 und am Übergang vom Boden 2 in die Seitenwand 4 der Flasche 1 haftende Kondensat auf die Seitenwand 4. Nach etwa einer halben bis einer Sekunde wird das Ventil 20 wieder geschlossen und gleichzeitig das Ventil 21 geöffnet, so daß nun ein fächer- oder scheibenförmiger Blasluftstrahl aus dem Ringspalt 17 der Blasdüse 15 austritt. Gleichzeitig wird die Blasdüse 15 während einer Dauer von etwa 1 Sekunde aus dem Flascheninneren herausgezogen. Dabei drängt die scheibenförmige Luftströmung, die an der Seitenwand 4 der Flasche 1 abgelenkt wird, die zuvor vom Boden 2 verdrängte Kondensatansammlung nach unten und rakelt das an der Seitenwand 4 haftende Kondensat ebenfalls nach unten (Figur 3 und 4) und treibt es schließlich aus der Öffnung 3 der Flasche 1 heraus.

Die dazu verwendete, steril gefilterte Druckluft hat Raumtemperatur. Neben dem Heraustreiben des Kondensats hat die eingeführte Druckluft auch eine trocknende Wirkung, so daß nach dem Austreten der Blasdüse 15 aus der Flasche 1 der Innenraum der Flasche 1 vollständig trocken ist. Die Reinigung des Flascheninneren beruht bei der beschriebenen Behandlung darauf, daß der eingeführte Wasserdampf beim Kondensieren an der Innenwand der Flasche haftende Fremdteilchen und Verunreinigungen unterwandert und löst, die dann beim Ausrakeln mit Druckluft zusammen mit dem Kondensat weggetragen werden. Zugleich hat der Wasserdampf eine sterilisierende Wirkung, so daß bei der beschriebenen Behandlung eine Entkeimung für solche Mikroorganismen erzielt wird, die im PH-Bereich kleiner 4,5 relevant sind, also für Füllgüter wie Fruchtsäfte, Kompotte, Wein, Feinkost. Soll eine für empfindlichere Güter ausreichende Entkeimung vorgenommen werden, wird anschließend an das oben beschriebene Reinigungsverfahren ein an sich bekanntes thermisches, physikalisches oder chemisches Sterilisierverfahren angeschlossen.

Wird ein besonders hohes Reinigungs- und Entkeimungsergebnis bis zu 99,99 % und darüber angestrebt, so kann der oben beschriebene Vorgang auch mehrfach wiederholt werden. Eine mehrfache Folge aus Bedampfung und jeweils folgender Entfernung des verkeimten Kondensats mittels Blasluft ist wirksamer als eine Verlängerung der Bedampfungszeit, weil das verkeimte Kondensat jeweils abgeführt wird und der Dampfstrahl dann wieder auf eine geringer verkeimte und nicht von Kondensat geschützte Fläche trifft.

Ein sehr gutes Ergebnis kann auch erzielt werden, wenn für die Bedampfung eine Düse ähnlich der oben beschriebenen Blasdüse 15 benutzt wird. Hier reinigt der vertikale, axiale Strahl erst den Boden 2 der Flasche 1, worauf der horizontale Fächerstrahl sowohl beim Bestreichen der Seitenwand 4 vom Boden 2 zur Einfüllöffnung 3 hin eine Reinigung der Seitenwand 4 als auch ein Ausschieben des verkeimten Kondensats bewirkt. Auch hier lassen sich bei Wiederholung dieser Bedampfungs- und Dampfrakelungs-Kombination mit abschließender Luftrakelung Entkeimungsraten von mehr als 99,99 % erzielen. Eine dazu geeignete Vorrichtung benötigt nur wenige Stationen.

Es wurde schon erwähnt, daß sich an diese Intensivreinigung gegebenenfalls eine zusätzliche thermische, chemische oder physikalische Sterilisierung anschließen kann. Diese zusätzliche Behandlung kann in den Reinigungsvorgang auch integriert werden. So kann vor oder nach dem Abrakeln des Kondensats und Trocknen mit Blasluft eine Bedampfung im Überdruckbereich geschaltet werden. Da nur eine geringe Zusatzsterilisation erforderlich ist, genügt es, die Mündung der Flasche abzudichten und Satt-Dampf von einer Temperatur von etwa 130° C - 135° C, entsprechend einem Druck von 1,75 - 2 bar Überdruck, einzuleiten und für einige Sekunden wirken zu lassen. Die Vorwärmung der Flasche durch den vorausgegangenen Reinigungsvorgang verhindert einen Thermoschock. Eine andere Möglichkeit ist die Einleitung von Wasserstoffperoxid (H₂O₂) in vernebelter oder verdampfter Form, z.B. zwischen einem ersten und zweiten Bedampfungs- und Ausblasvorgang oder auch anfangs der zweiten Folge. Eine weitere Möglichkeit ist eine UV-Bestrahlung der durch Dampf gereinigten und wieder trocken gerakelten Innenflächen, z.B. durch taktgleiches Einführen von UV-Röhren in die Flasche, zusammen mit den Düsen.

Ergänzend wird bemerkt, daß vorsorglich auch die Außenseite der Flaschen 1 gereinigt wird. Beim Bedampfen der Innenseite wird gleichzeitig auch die Außenseite bedampft und dann das Kondensat mittels Luftschleiern abgerakelt.

## Patentansprüche

1. Verfahren zum Reinigen und Sterilisieren von Verpackungsbehältern wie Flaschen, Ampullen oder dergleichen, mittels eines in deren Innenraum eingeblasenen Gases, dadurch gekennzeichnet, daß zunächst das Innere der Behälter (1) mit Wasserdampf beaufschlagt wird, so daß sich durch Kondensation ein Wasserfilm auf der Wand niederschlägt, und darauf das Kondensat am Boden (2) beginnend und zur Einfüllöffnung (3) führend mit zunächst einem axial gegen die Bodenmitte gerichteten und darauf mit einem gegen die Seitenwand (4) gerichteten fächerförmigen, vom Boden (2) zur Einfüllöffnung (3) bewegten Blasluftstrahl abgerakelt wird.

2. Verfahren nach Anspruch 1, dadurch gekennzeichnet, daß die Behälter (1) mit ihrer Öffnung (3) nach unten weisend behandelt werden.

3. Verfahren nach Anspruch 1 oder 2, dadurch gekennzeichnet, daß die Bedampfung der Innenwandung der Behälter (1) mit einem gegen den Boden (2) gerichteten Dampfstrahl und einem gegen die Seitenwand (4) gerichteten fächerförmigen Dampfstrahl durchgeführt wird, der vom Boden (2) zur Einfüllöffnung (3) bewegt wird.

4. Verfahren nach einem der Ansprüche 1 bis 3, dadurch gekennzeichnet, daß der kombinierte Bedampfungs-Ausblasvorgang wiederholt wird.

5. Verfahren nach einem der Ansprüche 1 bis 4, dadurch gekennzeichnet, daß Dampf bei atmosphärischem Druck während einer Zeitdauer von 2 bis 5 Sekunden eingeblasen wird und daß anschließend das Kondensat während einer Zeitdauer von 1 bis 3 Sekunden mit Druckluft abgerakelt wird.

6. Verfahren nach einem der Ansprüche 1 bis 5, dadurch gekennzeichnet, daß sich an den kombinierten Bedampfungs-Ausblasvorgang zusätzlich ein thermischer, physikalischer oder chemischer Sterilisiervorgang anschließt oder in diesen integriert ist.

7. Verfahren nach Anspruch 4, 5 oder 6, dadurch gekennzeichnet, daß zwischen zwei kombinierten Bedampfungs-Ausblasvorgängen ein thermischer, physikalischer oder chemischer Sterilisiervorgang zwischengeschaltet wird.

8. Verfahren nach einem der Ansprüche 1 bis 7, dadurch gekennzeichnet, daß der Blasluftstrahl aus steriler Luft mit Raumtemperatur oder erhöhter Temperatur besteht.

9. Vorrichtung zum Durchführen des Verfahrens nach einem der Ansprüche 1 bis 7, mit einer ersten Düse (11; 12) zum Beaufschlagen der Behälter (1) mit Wasserdampf und mit einer in das Behälterinnere einführbaren zweiten Düse (15) zum Zuführen von Blasluft, dadurch gekennzeichnet, daß die zweite Düse (15) eine axiale Öffnung (16) und einen radialen Ringspalt (17) aufweist und daß der axialen Öffnung sowie dem radialen Ringspalt nacheinander Druckgas zugeführt wird und die zweite Düse (15) während des Zuführens von Druckluft zu dem Ringspalt (17) aus dem Behälter (1) herausgezogen wird.

10. Vorrichtung nach Anspruch 9, dadurch gekennzeichnet, daß die erste Düse (12) zum Beaufschlagen eines Behälters (1) mit Wasserdampf eine axiale Öffnung (13) und mehrere radiale Öffnungen (14) hat und während des Zuführens von Wasserdampf aus dem Behälter (1) herausgezogen wird.

11. Vorrichtung nach Anspruch 9, dadurch gekennzeichnet, daß die axiale Öffnung (16) und der radiale Ringspalt (17) der zweiten Düse (15) an einem Doppelrohr (18, 19) angeordnet sind, in dessen Kanäle über Schaltventile (20, 21) Druckluft zugeleitet wird.

12. Vorrichtung nach Anspruch 11, dadurch gekennzeichnet, daß der radiale Ringspalt (17) kegelmantelartig geneigt ist.

## Claims

1. Process for cleaning and sterilising packaging containers such as bottles, ampoules or the like by means of a gas blown into the interior space thereof, characterised in that first the interior of the containers (1) is treated with steam so that a water film precipitates on the wall through condensation, and then the condensate is stripped off beginning at the base (2) and leading to the filling opening (3) by, at first, a blown air jet directed axially against the base centre and then by a fan-shaped blown air jet directed against the side wall (4) and moved from the base (2) to the filling opening (3).

2. Process according to Claim 1, characterised in that the containers (1) are treated with their opening (3) directed downwards.

3. Process according to Claim 1 or 2, characterised in that the steam treatment of the interior wall of the containers (1) is carried out with a steam jet directed against the base (2) and a fan-shaped steam jet which is directed against the side wall (4) and is moved from the base (2) to the filling opening (3).

4. Process according to one of Claims 1 to 3, characterised in that the combined steam treatment/blowing out operation is repeated.

5. Process according to one of Claims 1 to 4, characterised in that steam is blown in at atmospheric pressure for a duration of 2 to 5 seconds and in that subsequently the condensate is stripped off by compressed air for a duration of 1 to 3 seconds.

6. Process according to one of Claims 1 to 5, characterised in that the combined steam treatment/blowing out operation is additionally followed by or incorporates a thermal, physical or chemical sterilisation operation.

7. Process according to Claim 4, 5 or 6, characterised in that a thermal, physical or chemical sterilisation operation is interposed between two combined steam treatment/blowing out operations.

8. Process according to one of Claims 1 to 7, characterised in that the blown air jet consists of sterile air at ambient temperature or elevated temperature.

9. Device for carrying out the process according to one of Claims 1 to 7, having a first nozzle (11; 12) for treatment of the containers (1) with steam and having a second nozzle (15) which can be inserted into the container interior for admission of blown air, characterised in that the second nozzle (15) has an axial opening (16) and a radial annular gap (17) and in that compressed gas is admitted successively into the axial opening as well as into the radial annular gap and the second nozzle (15) is removed from the container (1) during the admission of compressed air to the annular gap (17).

10. Device according to Claim 9, characterised in that the first nozzle (12) has an axial opening (13) and a plurality of radial openings (14) for treating a container (1) with steam and is removed from the container (1) during the admission of steam.

11. Device according to Claim 9, characterised in that the axial opening (16) and the radial annular gap (17) of the second nozzle (15) are disposed on a double tube (18, 19) into the channels of which compressed air is fed via on-off valves (20, 21).

12. Device according to Claim 11, characterised in that the radial annular gap (17) is inclined in the manner of an envelope of a cone.

## Revendications

1. Procédé de nettoyage et de stérilisation de récipients d'emballage tels que des bouteilles, des ampoules ou objets analogues, par soufflage de gaz à l'intérieur des récipients, caractérisé en ce que tout d'abord de la vapeur d'eau est injectée dans le récipient (1) de manière à créer par condensation sur la paroi interne un film d'eau qui va s'écoulant du fond (2) vers l'ouverture de remplissage (3) et que l'on soumet à l'action de raclage produite d'abord par un jet d'air axial soufflé en direction du milieu du fond du récipient, puis par un jet d'air soufflé en éventail en direction de la paroi latérale (4) en se déplaçant du fond (2) vers l'ouverture de remplissage (3).

2. Procédé selon la revendication 1, caractérisé en ce que le récipient (1) a son ouverture (3) dirigée vers le bas, pendant le traitement.

3. Procédé selon la revendication 1 ou 2, caractérisé en ce que le traitement à la vapeur de la paroi interne du récipient (1) est réalisé au moyen d'un jet dirigé vers le fond (2) et d'un jet en éventail dirigé vers la paroi latérale (4), ce dernier se déplaçant du fond (2) vers l'ouverture de remplissage (3).

4. Procédé selon l'une des revendications 1 à 3, caractérisé en ce qu'il comporte une répétition du cycle injection de vapeur-soufflage d'air.

5. Procédé selon l'une des revendications 1 à 4, caractérisé en ce que la vapeur est injectée, à la pression atmosphérique, pendant une durée de 2 à 5 secondes, puis le condensat est raclé à l'air comprimé, pendant une durée de 1 à 3 secondes.

6. Procédé selon l'une des revendications 1 à 5, caractérisé en ce que le cycle injection de vapeur-soufflage d'air est complété par une phase de stérilisation par la chaleur ou par des moyens physiques ou chimiques, faisant suite au cycle ou intégrée dans celui-ci.

7. Procédé selon la revendication 4, 5 ou 6, caractérisé en ce qu'une phase de stérilisation par la chaleur ou par des moyens physiques ou chimiques est intercalée entre deux cycles injection de vapeur-soufflage d'air.

8. Procédé selon l'une des revendications 1 à 7, caractérisé en ce que l'air de soufflage est de l'air stérilisé, à la température ambiante ou à une température plus élevée.

9. Dispositif de mise en oeuvre du procédé selon l'une des revendications 1 à 7, comportant une première buse (11, 12) d'injection de vapeur d'eau dans le récipient (1) et une seconde buse (15) pouvant être introduite à l'intérieur du récipient pour y amener de l'air de soufflage, caractérisé en ce que la seconde buse (15) présente un orifice axial (16) et une fente annulaire radiale (17), alimentés successivement, en air comprimé, la seconde buse (15) se déplaçant en direction de la sortie du récipient pendant l'alimentation de la fente annulaire (17).

10. Dispositif selon la revendication 9, caractérisé en ce que la première buse (12) servant à traiter à la vapeur le récipient (1) comporte un orifice axial (13) et plusieurs orifices radiaux (14), cette buse se déplaçant vers la sortie du récipient (1) pendant l'injection de vapeur.

11. Dispositif selon la revendication 9, caractérisé en ce que l'orifice axial (16) et la fente annulaire radiale (17) de la seconde buse (15) sont disposés sur un tube à double paroi (18, 19) dont les canaux sont alimentés en air comprimé par des soupapes de commande (20, 21).

12. Dispositif selon la revendication 11, caractérisé en ce que la fente annulaire (17) est inclinée selon une surface conique.
